# EUROPEAN PATENT SPECIFICATION

(11) **EP 1 933 840 B1**
(45) Date of publication and mention of the grant of the patent: **13.11.2013**
(21) Application number: 06765096.0
(22) Date of filing: 24.07.2006
(51) Int. Cl.: A61K 31/5375, A61Q 19/00, A61P 17/10

(54) **USE OF DELMOPINOL IN THE TREATMENT OF ACNE**
VERWENDUNG VON DELMOPINOL IN DER AKNEBEHANDLUNG
UTILISATION DE DELMOPINOL POUR LE TRAITEMENT DE L'ACNE

(30) Priority: 22.07.2005 GB 0515138
(43) Date of publication of application: 25.06.2008
(73) Proprietor: Sinclair Pharmaceuticals Limited, Chester Business Park Chester CH4 9QZ (GB)
(72) Inventor: LANE, Jonathan Douglas, Godalming, Surrey GU7 1XW (GB)
(74) Representative: Jappy, John William Graham
(86) International application number: PCT/GB2006/002770
(87) International publication number: WO 2007/010294

(56) References cited:
- WO-A-92/08442
- US-A- 5 082 653
- ATTSTROM C B ET AL: "SHORT-TERM EFFECT OF TOPICAL APPLICATION OF DELMOPINOL ON SALIVARY MICROBIOLOGY, PLAQUE, AND GINGIVITIS" SCANDINAVIAN JOURNAL OF DENTAL RESEARCH, COPENHAGEN, DK, vol. 102, no. 1, 1994, pages 17-25, XP008067770 ISSN: 0029-845X

## Description

### Field of the invention

The present invention relates to the treatment of acne.

### Background to the invention

Acne is the general term used to refer to a number of skin disorders characterised by irritation of pilosebaceous units in the skin (Krautheim and Gollnick, Clinics in Dermatology, 2004; 22:398-407).

Acne is a common skin complaint that affects many people at some point in their lives. Acne occurs at hair follicles, which are cavities in the surface of the skin containing hairs that extend from the deep layers of the skin to protrude through pores at the surface. A sebaceous gland secretes sebum into the hair follicle, which drains to the skin surface to provide lubrication and prevent the skin from drying out.

Acne occurs when the pore is blocked (for example by dead skin cells or through excessive production of sebum) so that, instead of draining onto the skin surface, the sebum collects in the follicle. The blocked follicle may then become infected with bacteria. Propionibacteria, that are normally found on the skin, are associated with the progression of acne, in particular *Propionibacterium acnes (P. acnes).*

Various types of acne are known and the most common form is Acne Vulgaris. In a mild form of Acne Vulgaris, the pore may expose a sebum plug, the surface of which turns black. This is known as a "blackhead" or "open comedone". In other mild cases, the pore remains closed to the external environment and the trapped sebum and bacteria remain just below the surface of the skin. This is known as a "whitehead" or "closed comedone". Whiteheads and blackheads are relatively minor non-inflammatory complaints and are primarily considered to be a cosmetic rather than a medical problem.

However, acne becomes more serious when the blocked follicle becomes inflamed leading to a swollen, reddened appearance from the surface, forming papules and pustules. It is thought that inflammation is induced mainly by an immunologic reaction to extracellular products of *P. acnes* that colonise the blocked pore (Zouboulis, Clinics in Dermatology,2004; 22:360-366). The inflamed follicle can rupture onto the skin forming pustule heads. In serious cases, an intensely inflamed follicle can rupture under the skin, forming nodules and cysts, leading to deep scarring. Acne conglobata is the term given to the most severe form of acne.

Acne is unsightly and can cause severe scarring, in particular on the face. In addition to these physical effects, acne can cause severe psychological effects such as low self-esteem and depression.

Many topical treatments are currently used to treat acne. These typically contain active ingredients that have antimicrobial properties. Benzoyl peroxide is an important example of such an antimicrobial agent. Antibiotics are also commonly used, both topically and systemically, which has resulted in the development of bacterial resistance (as discussed in "European Surveillance Study on the Antibiotic Susceptibility of Propionibacterium Acnes", C. Oprica and C.E. Nord; Clinical Microbiology and Infection, Volume 11. No. 3, March 2005, pages 204 to 213).

Although existing acne treatments are effective to some degree, there is room for improvement in this field and there remains a strong need for effective acne treatments.

WO92/08442 discloses the use of compositions comprising delmopinol in treatments to prevent plaque formation.

US5082653 discloses the use of compositions comprising delmopinol in anti-plaque and anti-gingivitis treatments.

### Summary of the invention

The present invention is based on the surprising discovery that delmopinol, and its derivatives, is useful in the treatment of acne.

According to a first aspect, the present invention relates to use of a morpholino compound having the general formula 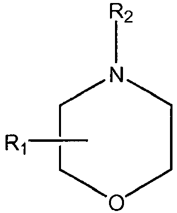 wherein R₁ is a straight or branched alkyl group containing 8 to 16 carbon atoms at the 2- or 3-position of the morpholino ring, and R₂ is a straight or branched alkyl group containing 2 to 10 carbon atoms, substituted with a hydroxy group except in the alpha-position, or pharmaceutically acceptable salts thereof, and an anti-inflammatory agent in the manufacture of a medicament for the treatment of acne.

According to a second aspect, the present invention relates to a composition comprising a morpholino compound as defined above and an anti-inflammatory agent for use in the treatment of acne.

### Description of the invention

The claimed morpholino compounds are beneficial in the treatment of acne.

As used herein, the term "acne" refers to any skin condition comprising a blocked pore of a pilosebaceous unit. The term "acne" includes the presence of white heads (closed comedones), blackheads (open comedones), papules, pustules, nodules and cysts. The term "acne" includes non-inflammatory acne, such as minor blackheads and whiteheads, and inflammatory acne wherein an immune response causes the blocked follicle to become inflamed, causing pustules, nodules or cysts. Preferably, the acne is characterised by the presence of *Propionibacterium acnes.*

In a preferred embodiment, the acne is Acne vulgaris. All forms of Acne vulgaris, from mild through moderate to severe, are within the scope of the invention. The severe forms of Acne vulgaris are sometimes referred to as Ance congloblata and Acne fulminans. Acne congloblata is the most severe form of acne vulgaris and is characterised by numerous lesions which are often connected. Acne fulminans is an acute onset version of Acne congloblata. The term "acne" also includes folliculitis, in particular gram-negative folliculitis, and pyoderma faciale, a type of facial acne that affects only females.

The claimed morpholino compounds are known *per se* as disclosed in US 4,894,221 and US 5,082,653.

The first aspect of the present invention relates to the use of the claimed compounds in the treatment of acne.

In the present invention, the morpholino compounds are defined according to the general formula shown above. In a preferred embodiment of the present invention, the sum of carbon atoms in the groups R₁ and R₂ is at least 10, and is preferably between 10 and 20. In a further preferred embodiment, the R₂ group terminates with the hydroxy group.

The preferred morpholino compound for use in the present invention is 3-(4-propyl-heptyl)-4-(2-hydoxyethyl) morpholine which is commonly known as Delmopinol (CAS No. 79874-76-3).

The morpholino compounds of the present invention can be used in their free base form or as a pharmaceutically acceptable salt thereof. Some examples of pharmaceutically acceptable salts are the salts of acids such as acetic acid, phosphoric, acid, boric acid, hydrochloric acid, maleic acid, benzoic acid, citric acid, malic acid, oxalic acid, tartaric acid, succinic acid, glutaric acid, gentisic acid, valeric acid, gallic acid, beta-resorcyclic acid, acetyl salicylic acid, salicylic acid, perchloric acid, barbituric acid, sulfanilic acid, phytic acid, p-nitro benzoic acid, stearic acid, palmitic acid, oleic acid, myristic acid, lauric acid and the like. The most preferred salt form is of hydrochloric acid. A preferred compound is delmopinol hydrochloride (CAS No. 98092-92-3).

The claimed compounds can be manufactured by any known method, for example, that disclosed in US 5,082,653 and WO 90/14342.

The claimed morpholino compounds are not thought to have any substantial antimicrobial action themselves. Therefore, the claimed compounds may advantageously be used in combination with a treatment that reduces the number of bacteria. For example, a composition containing the claimed morpholino compounds can be used as part of a skin cleansing routine.

A claimed morpholino compound is preferably used in combination with an antimicrobial agent. The antimicrobial agent can be any pharmaceutically acceptable compound which is effective against the bacteria associated with acne, for example, benzoyl peroxide.

Topical retinoids, which are thought to have an anti-inflammatory and indirect antibacterial effect, can also be used in combination with a claimed morpholino compound. Suitable retinoids will be apparent to the skilled person, and include tretinoin, isotretinoin, adapalene and tazortene.

An antibiotic may be used in a composition comprising a claimed morpholino compound. Any antibiotic that is effective against the bacteria associated with acne may be used. Preferably, antibiotics are selected from the group consisting of clindamycin, erythromycin, benzylpencillin, tetracycline, chloramphenicol, vancomycin and linezolid.

An anti-inflammatory agent may be used in a composition comprising a claimed morpholino compound. The skilled person will realise that the use of an anti-inflammatory agent is particularly useful in the treatment of inflammatory acne. Steroidal anti-inflammatories such as cortisone, or non-steroidal anti-inflammatories (NSAIDS), such as aspirin and ibuprofen, that inhibit cyclooxygenase isoenzymes are within the scope of the invention.

For the avoidance of doubt, all combinations of each of the composition ingredients described herein are within the scope of the invention.

The compositions for use in the present invention should be suitable for topical application to the skin and consist of the claimed compound in a pharmaceutically acceptable cream, ointment or gel. The composition can be hydrophillic or hydrophobic. The composition can be an aqueous composition, although other suitable solvents, such as alcohols or other organic solvents, may be used. A combination of solvents may also be used.

A suitable cream may be prepared by incorporating the active compound in a topical vehicle such as light liquid paraffin, dispersed in a aqueous medium using surfactants. An ointment may be prepared by mixing the active compound with a topical vehicle such as a mineral oil or wax. A gel may be prepared by mixing the active compound with a topical vehicle comprising a gelling agent.

Topically administrable compositions may also comprise a matrix in which a pharmaceutically active compound of the present invention is dispersed so that the compound is held in contact with the skin in order to administer the compounds transdermally.

The claimed morpholino compound should be included in the composition at a level at which it is effective which can be determined on the basis of the severity of the condition. The compound of the invention may be present in any suitable concentration. Typically, the compound is present in a concentration of from 0.01% (w/v) to 20% (w/v) e.g. 15% (w/v), preferably from 0.01 % (w/v) to 10% (w/v), preferably from 0.1 % (w/v) to 5% (w/v) and most preferably from 1% (w/v) to 3% (w/v) e.g. 2% (w/v). In general, a higher level of morpholino compound will be suitable for use in the medical treatment of acne than for the cosmetic use of improving the appearance of the skin. A suitable level can easily be selected by a person skilled in the art.

Where an antimicrobial, topical retinoid, antibiotic, anti-inflammatory agent and/or other additional component is included in the composition, these are also included at an effective level which can easily be determined by a person skilled in the art.

## Claims

1. Use of a composition comprising morpholino compound having the general formula 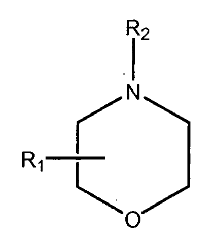 wherein R₁ is a straight or branched alkyl group containing 8 to 16 carbon atoms at the 2- or 3-position of the morpholino ring, and R₂ is a straight or branched alkyl group containing 2 to 10 carbon atoms, substituted with a hydroxy group except in the alpha-position, or pharmaceutically acceptable salts thereof, and an anti inflammatory agent in the manufacture of a medicament for the treatment of acne.

2. Use of a morpholino compound according to claim 1 wherein the sum of the carbon atoms in the groups R₁ and R₂ is at least 10, preferably between 10 and 20.

3. Use of a morpholino compound according to claim 1 or 2 wherein R₂ terminates with the hydroxy group.

4. Use of a morpholino compound according to any preceding claim wherein the morpholino compound is 3-(4-propyl-heptyl) -4-(2-hydroxyethyl) morpholino.

5. Use of a morpholino compound according to any preceding claim wherein the composition additionally comprises an antimicrobial agent.

6. Use of a morpholino compound according to claim 5 wherein the antimicrobial agent is an antibiotic, preferably an antibiotic selected from the group consisting of clindamycin, erythromycin, benzylpencillin, tetracycline, chloramphenicol, vancomycin and linezolid.

7. A composition comprising a morpholino compound as defined in claim 1 and an anti-inflammatory agent for use in the treatment of acne.

8. A composition for use according to claim 7, wherein the morpholino compound is as defined in any of claims 2 - 4.

9. A composition for use according to claim 7 or claim 8, further comprising an antimicrobial agent.

10. A composition for use according to claim 9, wherein the antimicrobial agent is as defined in claim 6.

## Patentansprüche

1. Verwendung einer Zusammensetzung, die eine Morpholinverbindung mit der allgemeinen Formel umfasst, 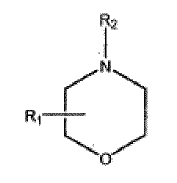 wobei R₁ eine gerade oder verzweigte Alkylgruppe ist, die 8 bis 16 Karbonatome an Position 2 oder 3 des Morpholinrings enthält, und R₂ eine gerade oder verzweigte Alkylgruppe, die 2 bis 10 Karbonatome enthält und mit einer Hydroxygruppe ersetzt wird, außer in der Alphaposition, oder pharmazeutisch akzeptierten Salzen davon, und einen entzündungshemmenden Wirkstoff bei der Herstellung eines Medikaments in der Aknebehandlung.

2. Verwendung einer Morpholinverbindung nach Anspruch 1, wobei die Summe der Karbonatome in den Gruppen R₁ und R₂ wenigstens 10, vorzugsweise aber zwischen 10 und 20 ist.

3. Verwendung einer Morpholinverbindung nach Anspruch 1 oder 2, wobei R₂ mit der Hydroxygruppe endet.

4. Verwendung einer Morpholinverbindung nach einem vorangehenden Anspruch, wobei die Morpholinverbindung 3-(4-Propylheptyl) -4-(2-Hydroxyethyl)morpholin ist.

5. Verwendung einer Morpholinverbindung nach einem vorangehenden Anspruch, wobei die Zusammensetzung zusätzlich einen antimikrobiellen Wirkstoff umfasst.

6. Verwendung einer Morpholinverbindung nach Anspruch 5, wobei der antimikrobielle Wirkstoff ein Antibiotikum ist, vorzugsweise ein Antibiotikum, das von der Gruppe ausgewählt wird, die aus Clindamycin, Erythromycin, Benzylpenicillin, Tetracyclin, Chloramphenicol, Vancomycin und Linezolid besteht.

7. Zusammensetzung, die eine Morpholinverbindung umfasst, wie in Anspruch 1 definiert, und einen entzündungshemmenden Wirkstoff zur Verwendung in der Aknebehandlung.

8. Zusammensetzung zur Verwendung nach Anspruch 7, wobei die Morpholinverbindung wie in einem der Ansprüche 2 - 4 definiert ist.

9. Zusammensetzung zur Verwendung nach Anspruch 7 oder Anspruch 8, die ferner einen antimikrobiellen Wirkstoff umfasst.

10. Zusammensetzung zur Verwendung nach Anspruch 9, wobei der antimikrobielle Wirkstoff wie in Anspruch 6 definiert ist.

## Revendications

1. Utilisation d'une composition comprenant un composé morpholino de la formule générale 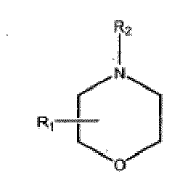 dans laquelle R₁ représente un groupe alkyle linéaire ou ramifié portant 8 à 16 atomes de carbone à la position 2 ou 3 du cycle morpholino, et R₂ représente un groupe alkyle linéaire ou ramifié portant 2 à 10 atomes de carbone, substitué par un groupe hydroxy excepté à la position alpha, ou des sels pharmaceutiquement acceptables de ceux-ci, et un agent anti-inflammatoire dans la fabrication d'un médicament pour le traitement de l'acné.

2. Utilisation d'un composé morpholino selon la revendication 1, dans laquelle la somme des atomes de carbone dans les groupes R₁ et R₂ est d'au moins 10, de préférence comprise entre 10 et 20.

3. Utilisation d'un composé morpholino selon la revendication 1 ou 2, dans laquelle R₂ se termine par le groupe hydroxy.

4. Utilisation d'un composé morpholino selon l'une quelconque des revendications précédentes, dans laquelle le composé morpholino est 3-(4-propyle-heptyle) -4-(2-hydroxyéthyle) morpholino.

5. Utilisation d'un composé morpholino selon l'une quelconque des revendications précédentes, dans laquelle la composition comprend en outre un agent antimicrobien.

6. Utilisation d'un composé morpholino selon la revendication 5, dans laquelle l'agent antimicrobien est un antibiotique, de préférence un antibiotique sélectionné parmi le groupe constitué de clindamycine, érythromycine, benzylpénicilline, tétracycline, chloramphénicol, vancomycine et linézolide.

7. Composition comprenant un composé morpholino tel que défini dans la revendication 1 et un agent anti-inflammatoire destiné à être utilisé dans le traitement de l'acné.

8. Composition destinée à être utilisée selon le mode opératoire de la revendication 7, dans laquelle le composé morpholino est tel que défini dans l'une quelconque des revendications 2 à 4.

9. Composition destinée à être utilisée selon le mode opératoire de la revendication 7 ou de la revendication 8, comprenant en outre un agent antimicrobien.

10. Composition destinée à être utilisée selon le mode opératoire de la revendication 9, dans laquelle l'agent antimicrobien est tel que défini dans la revendication 6.
